# EUROPEAN PATENT APPLICATION

(11) **EP 1 464 698 A1**
(43) Date of publication of application: **06.10.2004**
(21) Application number: 02783729.3
(22) Date of filing: 02.12.2002
(51) Int. Cl.: C12N 5/08

(54) **HUMAN CELL CULTURE MEDIUM AND CULTURE METHOD**

(30) Priority: 13.12.2001 JP 2001380426
(71) Applicant: Japan Science and Technology Agency, Kawaguchi-shi, Saitama 332-0012 (JP)
(72) Inventor: TAKAGI, Mutsumi, Ibaraki-shi, Osaka 567-0046 (JP); YOSHIDA, Toshiomi, Suita-shi, Osaka 565-0824 (JP); WAKITANI, Shigeyuki, Matsumoto-shi, Nagano 390-0304 (JP)
(74) Representative: Owen, Deborah Jane
(86) International application number: PCT/JP2002/012581
(87) International publication number: WO 2003/050273

(57) **Abstract**

A medium for human cells growing characterized by containing human serum and growth factors, and a method of growing human cells characterized by inoculating the medium with human cells and then growing the cells therein.

## Description

### Technical Field

The invention of this application relates to a medium for conveniently and efficiently growing human-derived cells useful in the field of regenerative medical techniques such as tissue regeneration and to a method for growing the human-derived cells using the medium.

### Background Art

Many fundamental findings of tissue regeneration using human cells have recently been made, and clinical applications of the findings are expected. But, in general cell cultures, particularly in the case of conducting a cell proliferation, a medium prepared by using a basal medium consisting of amino acids, vitamins, saccharides, and inorganic salts and adding to the basal medium 10-20% of bovine serum, particularly fetal bovine serum, as a cell growth factor is used. In addition, the fetal bovine serum is known to have remarkably higher cell growth activation as compared to adult bovine serum. However, the bovine serum cannot be mass-produced, is very expensive, and has a great individual difference (lot differences) in composition. Since an amount of the bovine serum per one lot is limited, it is necessary to perform complicated operations such as inspection of the lots and adjustment and management of the culture settings every time the lots are changed. Further, since the serum is a mixture containing physiologically active substances produced by blood cells and vascular endothelial cells, the serum has problems such as contamination by unknown virus and mycoplasma virus infection and makes it difficult to maintain a stable medium quality. In the medium preparation where the strict quality control is required, the above problems are considered very grave. Furthermore, possible contamination by an unknown pathogen such as the cow-derived prion from cow organs, tissues, and serums has become an issue. For the above reasons, in the human cell growth and culture for the tissue regeneration, there is a demand for development of a medium which does not contain heterozoic serum such as the bovine serum. However, in a culture without serum, it is difficult to achieve a cell growth to the extent achieved by the culture with serum. Also, various additives which can be substituted for a part of the growth activation effect of serum have been developed. Though it is better to contain the additives substituted for the serum effect in an amount as small as possible in view of the above-mentioned quality control, it is undesirably necessary to mix from a several to more than 10 serum substitutional additives in the actual use. Further, though human serum is congener to that of a patient to whom the tissue regeneration is adopted and remarkably less problematic in terms of the quality, it is significantly difficult to use fetal human serum for such purpose from ethical and social point of view, and adult human serum is not practical since it does not exhibit sufficient cell growth activation in spite of various factors contained therein. Considering clinical point of view such as tissue compatibility and probability of rejection, it is desirable to use serum (hereinafter referred to as autoserum) obtained from an individual from whom the human cells are obtained among the adult human serums. However, since the growth activation of serum on cells has the large individual difference (lot difference), as is the case with the fetal bovine serum, serums of plural lots are examined for the growth activation on cells so as to use the serum having the highest growth activation. The growth activation is not exhibited at all depending on the individual in the case of using the human adult serum, and, in the case of using the autoserum, the cell growth might not be achieved at all with the use of the autoserum because it is impossible to select the individual from whom the serum is obtained. Further, in the case of using the fetal bovine serum, no limitation is imposed on a dose in effect because the size of 1 lot is usually about 100 L, and it is possible to achieve the growth activation by increasing a concentration of the serum to be added to the medium when the growth activation is insufficient. However, in the case of using the human serum, particularly when using the autoserum, since an obtainable amount is not more than a several hundreds of milliliters, the concentration of the serum to be added to the medium cannot be increased easily even when the growth activation is insufficient.

In the case of culturing mesenchymal cells derived from bone marrow or cord blood, the mesenchymal cells are generally separated from a cell suspension of the bone marrow or the cord blood by a density gradient centrifugation using Ficoll solution to be used for inoculation and growth. In turn, a method for growing the mesenchymal cells by directly inoculating the bone marrow or the cord blood into a culture vessel without the separation step is under development. Though the direct culture method is more convenient and practical, it is remarkably difficult to grow the mesenchymal cells with the method due to contamination by a large amount of blood cells other than the mesenchymal cells and the like.

The invention of this application has been accomplished in view of the problems of the above-described conventional technologies, and an object thereof is to provide a medium for growing human cells comprising novel compositions.

Another object of the invention of this application is to provide a method of growing human cells using the novel medium.

### Disclosure of Invention

The inventors of this invention have conducted extensive researches to solve the above problems and have found that human serum obtained from any individual promotes a growth of human cells when a small amount of the serum is used in combination with growth factors.

This invention provides, as a first invention, a medium for growing human cells, the medium being characterized by comprising human serum and growth factors.

According to a preferred mode of the first invention, at least one species selected from the group consisting of human ectodermal cells, human mesodermal cells, human endodermal cells, human embryo-stem cells, human somatic stem cells, and cells included in a process of differentiation of fertilized human eggs into the above cells are used as the human cells. The human ectodermal cells may preferably be human nerve cells. The human mesodermal cells may preferably be at least one species selected from the group consisting of human vascular cells, human hemopoietic cells, and human mesenchymal cells. The human endodermal cells may preferably be at least one species selected from the group consisting of human hepatocytes, human hepatocytes, and human cystic cells. According to a preferred mode of the first invention, the growth factor is at least one species selected from the group consisting of nerve cell growth factor, hepatocyte growth factor, epidermal growth factor, thrombopoietin, stem cell factor, and fibroblast growth factor, and the human serum is serum obtained from an individual from whom the human cells to be cultured are obtained.

The invention of this application further provides, as a second invention, a method for growing human cells, the method being characterized by inoculating the medium of the first invention with the human cells and then growing the cells in the medium. According to a preferred mode of the growing method of the second invention, human bone marrow-derived mesenchymal cells or human cord blood-derived mesenchymal cells are grown. Further, according to another preferred mode of the method of the second invention, the medium is inoculated directly with tissue cells containing the human cells without subjecting the tissue cells to separation operation and then the human cells are grown in the medium.

### Best Mode for Carrying out the Invention

A medium of the first invention is prepared by adding, to a standard medium containing growth factors and nutrients used for supporting a cell growth and cell maintenance, at least human serum and growth factors.

As the standard medium, Iscove medium, RPMI medium, Dulbecco MEM medium, MEM medium, F12 medium and like mediums which are used for general animal cell growth and include no serum are usable. Also, factors other than the serum, which are disclosed in heretofore known literatures and the like as substances effective for growing or maintaining cells, such as fat and fatty acid sources, cholesterol, pyruvate, glucocorticoid, DNA and RNA synthetic nucleosides, and the like may be added to the medium.

As the human serum, human serum obtained from any part of the body may be used. Examples of the usable human serum are those separated from blood obtained from peripheral blood, bone marrow, cord blood, and the like. A human individual from whom the serum is obtained may be the person to whom cultured and grown human cells are to be grafted or other person, and it is needless to say that the ethical care such as satisfactory informed consent is required for the serum collection. Further, it is necessary to fully consider a health condition of a donor of the serum in the scientifically and ethically allowable range. Details of the above cautions are according to related laws. From the view point of tissue compatibility in grafting the cultured human cells or grafting regenerated tissue prepared by using the human cells, the serum is desirably autoserum obtained from the patient himself/herself. However, as to the growth activation of human cells, serum obtained from other person or that from the patient may be used.

As the growth factor, extracellular matrix components such as basic fibroblast growth factor, epithelium growth factor, platelet-derived growth factor, transferrin, interleukin-1, interleukin-2, interleukin-3, interleukin-4, interleukin-5, interleukin-6, interleukin-7, interleukin-8, interleukin-9, interleukin-10, interleukin-11, interleukin-12, interleukin-13, receptors of these interleukins, granulocyte-colony stimulating factor, granulocyte-colony stimulating factor receptor, erythropoietin, erythropoietin receptor, colony stimulating factor-1, macrophage colony stimulating factor, colony stimulating factor-1 receptor, stem cell factor, stem cell factor receptor, Flt-3 ligand, thrombopoietin, thrombopoietin receptor, epidermal growth factor, epidermal growth factor receptor, transforming growth factor, transforming growth factor receptor, diphtherotoxin receptor, epiregulin, neuregulin-1, neuregulin-2, neuregulin-3, platelet-derived growth factor receptor, acidic fibroblast growth factor, fibroblast growth factor receptor, insulin-like growth factor, insulin-like growth factor receptor, cell scattering factor, stem cell growth factor, stem cell growth factor receptor, vascular endothelial growth factor, vascular endothelial growth factor receptor, nerve growth factor, nerve growth factor receptor, glial cell line-derived neurotrophic factor, glial cell line-derived neurotrophic factor receptor, midkine, pleiotrophin, angiopoietin, betaglycan, endoglyn, activin, activin receptor, inhibin, bone morphogenetic protein, bone morphogenetic protein receptor, follistatin, noggin, chordin, smad, tumor necrosis factor, tumor necrosis factor receptor, lymphotoxin, Fas, Fas ligand, CD40, CD40 ligand, CD30, CD30 ligand, CD27, CD27 ligand, interferon-α, interferon-α receptor, interferon-β, interferon-β receptor, interferon-γ, interferon-γ receptor, serum albumin, insulin, collagen, fibronectin, and laminin can be used. Examples of the usable basic fibroblast growth factor (bFGF) include those obtained by a known isolation and purification method from an organ such as hypophysis, brain, retina, yellow body, adrenal, renal, placenta, prostate, thymus gland, and the like; those manufactured by a gene engineering method such as a recombinant DNA technology; and those obtained by modifying the above basic fibroblast growth factors to act as the basic fibroblast growth factor. Examples of the modified bFGFs are those obtained by modifying an amino acid sequence of any of the bFGFs obtained from organ by known isolation and purification method or the recombinant bFGFs manufactured by gene engineering method by adding at least one amino acid to the amino acid sequence, by deleting at least one amino acid from the amino acid sequence, or by substituting at least one amino acid of the amino acid sequence with another amino acid. In addition, in the protein manufacture by gene engineering method, a protein expressed in transformed cells is subject to various modifications in the cells after translation (the modification after translation) in some cases. Examples of the modification after translation are N-terminus methionine removal, N-terminus acetylation, sugar chain addition, limited decomposition by intracellular protease, myristoylation, isoprenylation, phosphorylation, and the like. Therefore, the bFGFs modified due to the modification after translation are included in the range of the modified bFGFs. Further, among the above growth factors, when the type of the growth factor varies depending on the types of animal, it is desirable to use the human growth factor even if growth activation of the human growth factor is identical with those of other animals.

A method of the second invention is characterized by inoculating the medium of the first invention with human cells and growing the cells in the medium.

The human cells which are to be cultured may be any human-derived cells or tissues, and examples thereof are human ectodermal cells, human mesodermal cells, human endodermal cells, cells included in a process of differentiation of fertilized human eggs into the above cells, human embryo-stem cells, human somatic stem cells, and the like.

In the invention of this application, examples of the human ectodermal cells are neuron cells, astrocyte cells, oligodendorocyte cells, human nerve cells which are stem cells of these cells, and the like, and the human ectodermal cells means cells and stem cells included in ectodermal tissues in the histologic sense. Examples of the human mesodermal cells are human vascular cells, human hemopoietic cells, human mesenchymal cells, and the like, and the human mesodermal cells means cells and stem cells included in mesodermal tissues in the histologic sense. Examples of the human hemopoietic cells are hemopoietic stem cells, hemopoietic precursor cells, erythroid cells, lymphocytes, granulocytes, platelet cells, and the like. The mesenchymal cells means, in the histologic sense, cells of connective tissues, such as osteocytes, chondrocytes, myocytes, cardiac myocytes, tendon cells, adipocytes, hair papilla cells, and dental pulp cells and cells capable of differentiating into these cells. Examples of cell forms are fibroblast, adipocyte, and the like. Examples of tissues in which the mesenchymal cells exist are bone, cartilage, muscle, cardiac muscle, tendon, adipose tissue, hair papilla, dental pulp, and the like. Further, the mesenchymal cells exist in and around solid organs such as vain, liver, and pancreas as well as in bone marrow and umbilical cord. There are may cells (mesenchymal stem cells) having multipotency of differentiating into various connective tissue cells in bone marrow, and it has been reported that CD105 antigen is specifically expressed on a surface of the mesenchymal stem cell. Therefore, the CD105 antigen is included in the mesenchymal cells in the method of this invention. In addition, in the case of growing the mesenchymal cells derived from bone marrow or cord blood, the mesenchymal cells may be separated from a cell suspension of the bone marrow or the cord blood by a density gradient centrifugation method using Ficoll solution according to a known method and then inoculated to be grown.

Examples of the human endodermal cells are hepatocytes, hepatic cells that are stem cells of the hepatocytes, pancreatic exocrine cells, pancreatic endocrine cells, hepatic cells that are stem cells of the pancreatic cells, cystic cells, and the like, and the human endodermal cells differentiate into liver and pancreas mainly. The human endodermal cells mean, in the histologic sense, cells and stem cells included in endodermal tissues.

As used herein, the growth in this invention relates to a change in the number of cells during culture and indicates a growth in the number of cells with time. Preferably, the time required for the number of cells in a certain cell population to be doubled is in the range of 1-150 hours.

### Examples

Hereinafter, the invention of this application will be described in more details and specifically in conjunction with examples; however, this invention is not limited by the following examples.

### (Example 1)

After receiving informed consents, 13 mL of a bone marrow was obtained from each of male and female who are three healthy volunteers, and then nuclear cell count of each of the bone marrows was performed with Türk's solution in accordance with a known method. Using 5 types of mediums shown in Table 1, each of the bone marrows was inoculated into cell culture dishes (product of Sumitomo Bakelite Co., Ltd.; bottom area of 1.8 cm², culture solution amount of 1 mL) in such a manner that a cell concentration is 6.0 × 10⁵ cell/cm², followed by a stationary culture in an incubator at 37°C and 5% CO₂.

Components of the mediums are as follows.
· DMEM standard medium (product of Gibco Industries, Inc.; product No. 31600-34)
· Basic fibroblast growth factor (bFGF: product of Gibco Industries, Inc.; product No. 100-18B); 10 ng/mL
· 10% serum:
   - Fetal bovine serum (product of Gibco Industries, Inc.; product No. 26140-079); or
   - Human peripheral blood-derived serums obtained from the donors of the bone marrows

The mediums were exchanged a day after and 2 days after the start of the culture, and floating cells (blood cells and the like) other than adherent cells were removed to find a small amount of the adherent cells on a dish bottom of each of the mediums. After 19 days, it was confirmed with a microscope that the adherent cells had grown close to confluent, and then the adherent cells of each of the dishes were removed by the trypsin treatment, followed by a measurement of a density of live cells adhered to the bottom of each of the dishes using a trypan blue liquid.

Results of the measurements are as shown in Table 1. Unit of the values of the cell densities in Table 1 is × 10⁴ cells/cm². As is apparent from the results of Table 1, in the mesenchymal cells obtained from each of the donors, though a growth effect was recognized with the addition of bFGF to the non-serum mediums, a density of growth in each of the non-serum mediums was low (about 1/5 of the fetal bovine serum medium). In contrast, though growth activation of the human serum itself was lower than that of the fetal bovine serum, it was significantly higher than that of the non-serum. Further, it was confirmed that growth activation stronger than that of the fetal bovine serum was achieved by adding bFGF to the human serum.

**Table 1**

| | | Donor Number | | |
|---|---|---|---|---|
| Serum | bFGF Addition | 1 | 2 | 3 |
| Fetal bovine serum | - | 1.52 | 5.80 | 2.50 |
| Non-Serum | - | 0.05 | 0.52 | 0.07 |
| Non-Serum | + | 0.28 | 1.03 | 0.47 |
| Human Serum | - | 0.39 | 3.60 | 0.12 |
| Human Serum | + | 4.07 | 11.28 | 3.50 |

### (Example 2)

HepG2 cells, Huh7 cells, and human primary hepatocytes (product of Asahi Techno Glass Corporation) as well as 5 types of mediums shown in Table 2 were used as human liver cells. Each species of the cells was inoculated into cell culture dishes (product of Sumitomo Bakelite Co., Ltd.; bottom area of 2.1 cm²; culture solution amount of 5 mL) in such a manner that a cell concentration of each of HepG2 cells and Huh7 cells was 1.0 × 10⁴ and a cell concentration of the human primary hepatocytes was 1.0 × 10⁵ cell/cm², followed by a stationary culture in an incubator at 37°C and

### 5%CO₂.

Components of the mediums are as follows.
· DMEM standard medium (product of Gibco Industries, Inc.; product No. 31600-34)
· L-proline: 30 µg/mL
· Insulin: 0.5 µg/mL
· L-ascorbic acid phosphate ester: 0.2 mM
· Hepatocyte growth factor (HGF): 10 ng/mL
· Epidermal growth factor (EGF): 10 ng/mL
· 10% serum:
   - Fetal bovine serum (product of Gibco Industries, Inc.; product No. 26140-079); or
   - Human peripheral blood-derived serums

A density of adherent cells in each of the dishes of each of HepG2 cells and Huh7 cells was measured by enucleation and staining 8 days after confirming using a microscope that the adherent cells had grown. A density of adherent cells of each of the dishes of the primary hepatocytes was measured in the same manner 14 days after the confirmation of a growth of the adherent cells using microscope.

Results are as shown in Table 2. Unit of the values of the cell densities in Table 2 is ×10⁴ cells/cm². From Table 2, it is apparent that little growth effect was achieved by adding HGF and EGF to the non-serum mediums of HepG2 cells and Huh7 cells. In contrast, though growth activation of the human serum itself was lower than that of the fetal bovine serum, it was significantly higher than that of the non-serum. Further, it was confirmed that growth activation stronger than that of the fetal bovine serum was achieved by adding HGF and EGF to the human serum in each species of the hepatocytes.

**Table 2**

| | | Hepatocytes | | |
|---|---|---|---|---|
| Serum | HGF and EGF Addition | HepG2 Cells | Huh7 Cells | Primary Hepatocytes |
| Fetal bovine serum | - | 20.5 | 15.0 | 19.5 |
| Non-Serum | - | 1.1 | 1.5 | 10.7 |
| Non-Serum | + | 1.2 | 1.7 | 12.4 |
| Human Serum | - | 11.8 | 9.8 | 15.2 |
| Human Serum | + | 22.5 | 17.0 | 23.0 |

### (Example 3)

Human nerve astrocyte cells (NHA; product of Clonetics Cell Systems) and human nerve precursor cells (NHMP; product of Clonetics Cell Systems) as well as 5 types of mediums shown in Table 3 were used. Each species of the cells was inoculated into cell culture dishes (product of Sumitomo Bakelite Co., Ltd.; bottom area of 1.8 cm²; culture solution amount of 2 mL) in such a manner that a cell concentration of each species of the cells was 2.0 × 10³ cell/cm², followed by a stationary culture in an incubator at 37°C and 5%CO₂.

Components of the mediums are as follows.
· DMEM standard medium (product of Gibco Industries, Inc.; product No. 31600-34)
· Nerve cell growth factor (NGF): 100 ng/mL
· 10% serum:
   - Fetal bovine serum (product of Gibco Industries, Inc.; product No. 26140-079); or
   - Human peripheral blood-derived serums

10 days after confirming using a microscope that the cells had grown, adherent cells of each of the dishes were removed by the trypsin treatment, followed by a measurement of a density of live cells adhered to the bottom of each of the dishes using a trypan blue liquid.

Results are as shown in Table 3. Unit of the values of the cell densities in Table 3 is ×10⁴ cells/cm². From the results shown in Table 3, it is apparent that little growth effect was achieved by adding NGF to the non-serum mediums of each of the nerve cells. In contrast, though growth activations of the human serum itself in the NHA cells and the NHMP cells were a little lower than and about 1/2 of that of the fetal bovine serum, they were significantly higher than that of the non-serum. Further, it was confirmed that growth activation stronger than that of the fetal bovine serum was achieved by adding NGF to the human serum.

**Table 3**

| | | Nerve Cells | |
|---|---|---|---|
| Serum | NGF Addition | NHA Cells | NHMP Cells |
| Fetal bovine serum | - | 1.05 | 2.13 |
| Non-Serum | - | 0.21 | 0.23 |
| Non-Serum | + | 0.21 | 0.21 |
| Human Serum | - | 0.83 | 0.95 |
| Human Serum | + | 1.50 | 2.76 |

### Industrial Applicability

As described in detail in the foregoing, according to this invention, the medium for growing human cells using human serum and the method of growing human cells are provided.

## Claims

1. A medium for growing human cells, **characterized by** comprising human serum and growth factors.

2. The medium according to claim 1, wherein at least one species selected from the group consisting of human ectodermal cells, human mesodermal cells, human endodermal cells, human embryo-stem cells, human somatic stem cells, and cells included in a process of differentiation of fertilized human eggs into the above cells is used as the human cells.

3. The medium according to claim 2, wherein the human ectodermal cells are human nerve cells.

4. The medium according to claim 2, wherein at least one species selected from the group consisting of human vascular cells, human hemopoietic cells, and human mesenchymal cells is used as the human mesodermal cells.

5. The medium according to claim 2, wherein at least one species selected from the group consisting of human hepatocytes, human hepatocytes, and human cystic cells is used as the human endodermal cells.

6. The medium according to any one of claims 1 to 5, wherein the growth factor is at least one species selected from the group consisting of nerve cell growth factor, hepatocyte growth factor, epidermal growth factor, thrombopoietin, stem cell factor, and fibroblast growth factor.

7. The medium according to any one of claims 1 to 5, wherein the human serum is serum obtained from an individual from whom the human cells to be cultured are obtained.

8. A human cell growth method **characterized by** inoculating the medium defined in any one of claims 1 to 7 with human cells to grow the cells therein.

9. The method according to claim 8, comprising growing human bone marrow-derived mesenchymal cells.

10. The method according to claim 8, comprising growing human cord blood-derived mesenchymal cells.

11. The method according to any one of claims 8 to 10, comprising directly inoculating the medium with tissue cells including the human cells without subjecting the tissue cells to separation operation and growing the human cells in the medium.
